# EUROPEAN PATENT SPECIFICATION

(11) **EP 3 067 041 B1**
(45) Date of publication and mention of the grant of the patent: **04.12.2019**
(21) Application number: 14854824.1
(22) Date of filing: 16.10.2014
(51) Int. Cl.: A61K 8/66, A61K 8/64, A61K 8/81, A61K 8/84, A61Q 5/10

(54) **METHOD FOR DYEING KERATIN FIBERS, KERATIN FIBER DYE**
VERFAHREN ZUR FÄRBUNG KERATINISCHER FASERN, KERATINFASERFARBSTOFF
PROCÉDÉ POUR COLORER DES FIBRES DE KÉRATINE, COLORANT DE FIBRES DE KÉRATINE

(30) Priority: 17.10.2013 JP 2013216393
(43) Date of publication of application: 14.09.2016
(73) Proprietor: Shisei Chemical Co., Ltd., Tokorozawa-shi, Saitama 359-0024 (JP); Tsujino, Yoshio, Kobe-shi, Hyogo 658-0003 (JP)
(72) Inventor: TSUJINO Yoshio, Kobe-shi Hyogo 658-0003 (JP); OKAMURA Akinori, Sayama-shi Saitama 350-1335 (JP); SASAKI Ryoitsu, Sayama-shi Saitama 350-1335 (JP)
(74) Representative: Vossius & Partner Patentanwälte Rechtsanwälte mbB
(86) International application number: PCT/JP2014/077504
(87) International publication number: WO 2015/056730

(56) References cited:
- EP-A1- 1 142 562
- WO-A1-99/36035
- WO-A1-2006/036746
- WO-A2-02/39966
- JP-A- H07 316 029
- JP-A- 2002 509 087
- JP-A- 2006 282 512
- JP-A- 2008 513 509

## Description

### TECHNICAL FIELD

The present invention relates to a method for dyeing keratin fibers and a keratin fiber dye, and more particularly to a method for dyeing keratin fibers and a keratin fiber dye which have excellent dyeing properties and which result in less damage and irritation to the hair and scalp even when the head hair is dyed.

### BACKGROUND ART

Conventionally, as a keratin fiber dye such as a hair dye, there has been generally used an oxidation-type keratin fiber dye containing a diamine-based oxidation dye such as paraphenylenediamine and an oxidant such as hydrogen peroxide as major components. As the oxidant, hydrogen peroxide has been conventionally often used. However, since hydrogen peroxide has an extremely strong oxidation action, use of hydrogen peroxide together with an alkali results in damages to the hair and scalp when applied to the head hair. Also, the diamine-based oxidation dye acts as an allergen, so that, when the scalp is damaged, the diamine-based oxidation dye is liable to penetrate through the scalp, resulting in a problem of inducing serious irritation.

In recent years, in order to solve these problems, there has been proposed a keratin fiber dye in which laccase, which is one kind of multicopper oxidases, is blended in place of hydrogen peroxide (Patent Documents 1 and 2).

### PRIOR ART DOCUMENT

### PATENT DOCUMENTS

Patent Document 1: JP-T-2002-509091
Patent Document 2: JP-A-2002-47144

### SUMMARY OF THE INVENTION

### PROBLEMS TO BE SOLVED BY THE INVENTION

However, when a conventional oxidase is used as an oxidant of a head hair dye, the dyeing properties are inferior as compared with the case of using hydrogen peroxide, so that, in order to compensate for this, there is a need to blend the oxidation dye in a large amount. When the oxidation dye is used in a large amount, the diamine-based oxidation dye penetrates through the scalp even when there is no damage in the scalp. Eventually, the problem of irritation is not sufficiently solved.

Therefore, intensive researches have been made on the cause of low dyeing properties of a dye that uses an oxidase. It is said that keratin fibers of human head hair or the like has an isoelectric point of about 3.0 to 5.5 (typically around 3.7). The isoelectric point of an oxidase is of the same degree. However, the hair-dyeing step is performed in a pH range (about 6.5 to 11.0) higher than this value, so that both the keratin fibers and the oxidase are negatively charged, and this is supposed to give adverse effects on the dyeing properties.

In detail, when the two are charged to be strongly negative, the oxidase and the keratin fibers repulse each other in the dyeing step, so that the oxidase oxidizes the oxidation dye for generating color at a position distant from the keratin fibers. Eventually, it is supposed that the oxidation dye molecules that have generated color are distributed at a position located far away from the keratin fibers. Therefore, the oxidation dye that has generated color is less likely to be adsorbed and distributed to the keratin fibers, and it is presumed that, when the chemical liquid is washed away after the dyeing, many of the oxidation dye molecules that have generated color are washed away.

As a result of intensive researches in order to solve the aforementioned problems, the present inventors have obtained a finding that, when the electrostatic repulsive force between the oxidase and the keratin fibers is negated in the hair-dyeing step, the oxidase moves to a position close to the keratin fibers and the oxidation dye that has generated color is distributed in the neighborhood of the keratin fibers, so that the oxidation dye is more likely to be adsorbed and distributed to the keratin fibers, and the aforementioned problems can be solved. This finding has led to the completion of the present invention.

That is, an object of the present invention is to solve the aforementioned problems of the prior art and to provide a method for dyeing keratin fibers and a keratin fiber dye not only having excellent hair-dyeing properties but also which, by using a substance that is positively charged under the pH conditions during the dyeing as an electrostatic repulsion inhibitor, negate the electrostatic repulsive force between the oxidase and the keratin fibers, to thereby result in less damage and irritation to the hair and scalp at the time of use even when the dye is used for the head hair.

### SOLUTIONS TO THE PROBLEMS

In other words, the present invention is as defined in the claims. Meanwhile, the present disclosure may be summarized by the following items (1) to (10).
(1) A method for dyeing keratin fibers using an oxidation dye and an oxidase that can oxidize the oxidation dye and under pH conditions where both the oxidase and the keratin fibers are negatively charged, which comprises the step of:
   before dyeing, treating the keratin fibers with an electrostatic repulsion inhibitor having a property of being positively charged under the pH conditions during the dyeing.
(2) A method for dyeing keratin fibers using an oxidation dye and an oxidase that can oxidize the oxidation dye and under pH conditions where both the oxidase and the keratin fibers are negatively charged, which comprises the step of:
   using an agent containing at least an electrostatic repulsion inhibitor having a property of being positively charged under the pH conditions during the dyeing and the oxidase.
(3) A method for dyeing keratin fibers using an oxidation dye and an oxidase that can oxidize the oxidation dye and under pH conditions where both the oxidase and the keratin fibers are negatively charged, which comprises the steps of:
   before dyeing, treating the keratin fibers with an electrostatic repulsion inhibitor having a property of being positively charged under the pH conditions during the dyeing, and
   using an agent containing at least the electrostatic repulsion inhibitor and the oxidase.
(4) The method for dyeing keratin fibers according to anyone of claims 1 to 3, wherein the electrostatic repulsion inhibitor is at least one kind selected from the group consisting of ε-polylysine, polyquaternium-2, polyquaternium-7, and poly[oxyethylene(dimethylimino)propyl(dimethylimino)ethylene].
(5) A keratin fiber dye comprising an electrostatic repulsion inhibitor, an oxidation dye, and an oxidase that can oxidize the oxidation dye,
   wherein, under pH conditions during dyeing, the oxidase has a property of being negatively charged, and
   wherein the electrostatic repulsion inhibitor has a property of being positively charged.
(6) The keratin fiber dye according to claim 5, which is a one-agent type containing the electrostatic repulsion inhibitor, the oxidation dye, and the oxidase.
(7) The keratin fiber dye according to claim 7, which is a two-agent type where the electrostatic repulsion inhibitor is contained separately from the oxidation dye and the oxidase.
(8) The keratin fiber dye according to claim 5, which is a two-agent type where the electrostatic repulsion inhibitor and the oxidation dye are contained separately from the oxidase.
(9) The keratin fiber dye according to claim 5, which is a two-agent type where the electrostatic repulsion inhibitor and the oxidase are contained separately from the oxidation dye.
(10) The keratin fiber dye according to claim 5, which is a three-agent type where the electrostatic repulsion inhibitor, the oxidation dye, and the oxidase are contained separately.

### ADVANTAGES OF THE INVENTION

According to the present invention, when keratin fibers are dyed under pH conditions where both an oxidase and the keratin fibers are negatively charged, at least one of the oxidase and the keratin fibers is/are charged at a reduced amount or is/are positively charged to negate or reduce an electrostatic repulsive force, or conversely, an attractive force is generated, by treating the keratin fibers with an electrostatic repulsion inhibitor as defined in claim 1 having a property of being positively charged under the pH conditions during the dyeing, or by using an agent containing at least the oxidase as defined in claim 1 and the electrostatic repulsion inhibitor, or by performing both of these. Therefore, the oxidase and the keratin fibers do not repulse each other, or the oxidase and the keratin fibers attract each other in the dyeing step, so that the oxidase oxidizes and polymerizes the oxidation dye near the keratin fibers to generate color. For this reason, the oxidation dye that has generated color is liable to be distributed in the neighborhood of the keratin fibers and is liable to be adsorbed and distributed to the keratin fibers, so that the dyeing properties are improved.

### MODE FOR CARRYING OUT THE INVENTION

In the present invention, the keratin fibers include fibers that contain keratin as a major component, such as the hair or wool. Also, the hair includes not only the head hair but also a wig and the like made of the hair. In the following description, the hair (head hair) will be exemplified as the keratin fibers.

The method for dyeing keratin fibers according to the present invention is a method for dyeing keratin fibers using an oxidation dye and an oxidase that can oxidize the oxidation dye, wherein the electrostatic repulsion inhibitor and the oxidase are as defined in claim 1, and under pH conditions where both the oxidase and the keratin fibers are negatively charged, the method is characterized in that, before dyeing, the keratin fibers are treated with an electrostatic repulsion inhibitor having a property of being positively charged under the pH conditions during the dyeing, or an agent containing at least the oxidase and the electrostatic repulsion inhibitor is used, or both of these are performed.

The keratin fiber dye of the present invention contains an oxidation dye, an oxidase that can oxidize the oxidation dye, and an electrostatic repulsion inhibitor, wherein the electrostatic repulsion inhibitor and the oxidase are as defined in claim 1, and is characterized in that, under pH conditions during dyeing, the oxidase has a property of being negatively charged, and the electrostatic repulsion inhibitor has a property of being positively charged.

The oxidation dye used in the present invention may be of any kind as long as the oxidase can oxidize the oxidation dye, and examples thereof include phenylenediamines, indolines, indoles, phenols, catechols, hydroquinones, resorcins, triphenols, naphthalenes, and the like. Among these, phenylenediamines, indolines and indoles are preferable.

Examples of the paraphenylenediamines include paraphenylenediamine, 5-aminoorthocresol, orthoaminophenol, metaaminophenol, paraaminophenol, 2,4-diaminophenol, 2,6-diaminopyridine, 5-(2-hydroxyethylamino)-2-methylphenol, N,N-bis(β-hydroxy) -paraphenylenediamine sulfate, paranitro-orthophenylenediamine, sodium paranitro-2',4'-diaminoazobenzenesulfate, toluene-2,5-diamine, 5-aminoorthocresol sulfate, paraaminophenol sulfate, orthochloro-paraphenylenediamine sulfate, 4,4'-diaminodiphenylamine sulfate, paramethylaminophenol sulfate, paraphenylenediamine sulfate, metaphenylenediamine sulfate, toluene-2,5-diamine sulfate, 2,4-diaminophenoxyethanol hydrochloride, toluene-2,5-diamine hydrochloride, metaphenylenediamine hydrochloride, 2,4-diaminophenol hydrochloride, 3,3'-iminodiphenol, paraphenylenediamine hydrochloride, N-phenyl-paraphenylenediamine hydrochloride, N-phenyl-paraphenylenediamine acetate, 1,5-dihydroxynaphthalene, tolylene-3,4-diamine, paramethylaminophenol, N,N'-bis(4-aminophenyl)-2,5-diamino-1,4-quinonediimine, orthoaminophenol sulfate, 2,4-diaminophenol sulfate, metaaminophenol sulfate, and the like. Among these, paraphenylenediamine, paraaminophenol, orthoaminophenol, 2,4-diaminophenol, toluene-2,5-diamine and salts thereof are particularly preferable in view of substrate specificity. These are used either singly or in combination of two or more if necessary.

Examples of the indolines may include indoline, 5,6-dihydroxyindoline, N-methyl-5,6-dihydroxyindoline, N-ethyl-5,6-dihydroxyindoline, N-butyl-5,6-dihydroxyindoline, 4-hydroxy-5-methoxyindoline, 6-hydroxy-7-methoxyindoline, 6,7-dihydroxyindoline, 4,5-dihydroxyindoline, 4-methoxy-6-hydroxyindoline, N-hexyl-5,6-dihydroxyindoline, 2-methyl-5,6-dihydroxyindoline, 3-methyl-5,6-dihydroxyindoline, 4-hydroxyindoline,2,3-dimethyl-5,6-dihydroxyindoline, 2-methyl-5-ethyl-6-hydroxyindoline, 2-methyl-5-hydroxy-6-β-hydroxyethylindoline, 4-hydroxypropylindoline, 2-hydroxy-3-methoxyindoline, 6-hydroxy-5-methoxyindoline, 6-hydroxyindoline, 5-hydroxyindoline, 7-hydroxyindoline, 7-aminoindoline, 5-aminoindoline, 4-aminoindoline, 5,6-dihydroxyindolinecarboxylic acid, 1-methyl-5,6-dihydroxyindoline, salts thereof, and the like. These are used either singly or in combination of two or more if necessary.

Examples of the indoles may include 4,5-dihydroxyindole, 5,6-dihydroxyindole, 6,7-dihydroxyindole, N-methyl-5,6-dihydroxyindole, N-ethyl-5,6-dihydroxyindole, N-hexyl-5,6-dihydroxyindole, 2-methy1-5,6-dihydroxyindole, 3-methyl-5,6-dihydroxyindole, 4-hydroxyindole, 2,3-dimethyl-5,6-dihydroxyindole, 2-methyl-5-ethyl-6-hydroxyindole, 2-methy1-5-hydroxy-6-β-hydroxyethylindole, 4-hydroxypropylindole, 2-hydroxy-3-methoxyindole, 4-hydroxy-5-methoxyindole, 6-hydroxy-7-methoxyindole, 6-hydroxy-5-methoxyindole, 6-hydroxyindole, 5-hydroxyindole, 7-hydroxyindole, 7-aminoindole, 5-aminoindole, 4-aminoindole, 5,6-dihydroxyindolecarboxylic acid, 1-methyl-5,6-dihydroxyindole, salts thereof, and the like. These are used either singly or in combination of two or more if necessary.

Examples of the phenols include cresol, thymol, and the like; examples of the catechols include catechol, guaiacol, vanillin, caffeic acid, and the like; examples of the triphenols include pyrogallol, esters thereof, and the like. These are used either singly or in combination of two or more if necessary.

It is necessary that the oxidase that can be used in the present invention can oxidize the oxidation dye. Specific examples of the oxidase include multicopper oxidases such as copperefflux oxidase (CueO), laccase, bilirubin oxidase (BOD), and the like. In the present invention, the oxidase is bilirubin oxidase (BOD).

A suitable pH range of the above oxidase is typically about 7.0 to 7.5, so that a dyeing treatment is preferably performed under a pH range of this degree. However, the keratin fibers have an isoelectric point of about 3.0 to 5.5, and typically around 3.7. Therefore, when the dyeing treatment is performed under the above suitable pH range, the keratin fibers are negatively charged. Also, many oxidases that can oxidize the oxidation dye have a low isoelectric point (for example, the isoelectric point of a wild-type BOD derived from Myrothecium verrucaria is 4.1), so that, when the treatment is performed under the above suitable pH range, the oxidase is also negatively charged. In other words, when the dyeing treatment is performed under the suitable pH range of the oxidase, both the keratin fibers and the oxidase are negatively charged to generate an electrostatic repulsive force between the two, and this electrostatic repulsive force gives adverse effects on the dyeing properties.

The present invention is characterized in that this electrostatic repulsive force is removed by treatment with an electrostatic repulsion inhibitor. In the present invention, the electrostatic repulsion inhibitor refers to a substance having a property of being positively charged under the pH range where the dyeing treatment is performed. Specifically, an oligopeptide obtained by polymerization of basic amino acids, a cationic polymer that is conventionally blended in shampoo and hair conditioner products, other cationic polymers, or the like are examples of an electrostatic repulsion inhibitor.

In the present invention, the electrostatic repulsion inhibitor is selected from ε-polylysine, polyquaternium-2, polyquaternium-7, polyquaternium-72 and PA1.

Preferable examples of oligopeptide as the electrostatic repulsion inhibitor may include ε-polylysine and the like.

Also, examples of a cationic polymer as electrostatic repulsion inhibitor may include polyquaternium-2, polyquaternium-4, polyquaternium-6, polyquaternium-7, polyquaternium-10, polyquaternium-11, polyquaternium-22, polyquaternium-39, polyquaternium-51, polyquaternium-55, polyquaternium-64, polyquaternium-72, and the like.

Further, examples of other cationic polymers as electrostatic repulsion inhibitor include poly[oxyethylene(dimethylimino)propyl(dimethylimino)ethylene] (hereafter, this cationic polymer may be abbreviated as PA1) that has been developed as an enzyme stabilizer, and the like. The PA1 is represented by Chemical Formula (I) below. 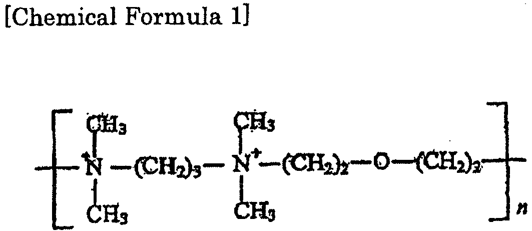 poly[oxyethylene(dimethylimino)propyl(dimethylimino)ethylene] (PA1, *M*_{w} = 63000)

These are used either singly or in combination of two or more if necessary.

In the present invention, among the electrostatic repulsion inhibitors as described above, those that are liable to be adsorbed onto the keratin fibers and can make the surface of the keratin fibers be positively charged when adsorbed are preferable. Such an electrostatic repulsion inhibitor can be evaluated and selected by a simple test using a basic dye.

That is, when the surface of the keratin fibers is positively charged by the electrostatic repulsion inhibitor, electrostatic repulsion is generated between the surface of the keratin fibers and the basic dye that is also positively charged, so that the keratin fibers are less likely to be dyed. Conversely stated, the electrostatic repulsion inhibitor such that the treated keratin fibers are less likely to be dyed by the basic dye makes the surface of the keratin fibers be positively charged. By utilizing this property, preferable electrostatic repulsion inhibitors can be evaluated and selected.

Hereafter, a method for evaluating an electrostatic repulsion inhibitor will be described with human hair as an example.

First, after a hair bundle of human white hair (100%) is immersed into a 0.5 wt% solution of an electrostatic repulsion inhibitor to be evaluated for 5 minutes, the hair bundle is washed with flowing water of 40°C for 30 seconds, followed by air-drying to obtain a treated hair bundle.

After this treated hair bundle is immersed into a 0.5 wt% basic blue 99 solution (pH is adjusted to 6.81) for 10 minutes (bath ratio of 1:10), the hair bundle is washed with flowing water of 40°C for 30 seconds, followed by air-drying to obtain a dyed hair bundle. A color difference A between the obtained dyed hair bundle and an untreated human white hair bundle in the Lab color representation system is measured.

The untreated human white hair bundle is also dyed in the same manner as described above, and a color difference B between the dyed hair bundle and the untreated human white hair bundle is measured.

By comparing the color difference A with the color difference B measured in the above-described manner, one having the color difference A being lower than the color difference B can be suitably used as the electrostatic repulsion inhibitor in the present invention. The difference between the color difference A and the color difference B (color difference B - color difference A) is preferably a positive value.

In the method for dyeing keratin fibers and the keratin fiber dye according to the present invention, dyes and pigments other than the oxidation dye can be used as well in accordance with the needs within a range that does not impair the effects of the present invention. Examples of these dyes and pigments may include tar pigments, HC dyes, basic dyes, direct dyes, and the like. These are used either singly or in combination of two or more if necessary. By incorporating these dyes and pigments, the hair can be dyed in various color tones.

Examples of the tar pigment include pigments that are designated by Ordinance No. 30 of Ministry of Health and Welfare issued on August 31, 1966 "Ministerial Ordinance that defines tar pigments that can be used in pharmaceuticals and others". Examples of the HC dyes include HC blue 2, HC orange 1, HC red 1, HC red 3, HC yellow 2, HC yellow 4, and the like; examples of the basic dyes include basic blue 99, basic brown 16, basic brown 17, basic red 51, basic red 76, basic yellow 57, and the like; and examples of the direct dyes include 2-amino-6-chloro-4-nitrophenol, 3-methylamino-4-nitrophenoxyethanol, 2-amino-3-nitrophenol, 4-hydroxypropylamino-3-nitrophenol, and the like. These are used either singly or in combination of two or more if necessary.

In the method for dyeing keratin fibers and the keratin fiber dye according to the present invention, various components that are ordinarily used in cosmetics and keratin fiber dyes can be used within a range that does not impair the effects of the present invention.

Examples of surfactants include cationic surfactants such as lauryltrimethylammonium chloride and stearyltrimethylammonium chloride; nonionic surfactants such as polyoxyethylenecetylether, polyoxyethyleneoleylether, polyoxyethylenebehenylether, polyoxyethylene castor oil, polyoxyethylene hardened castor oil, and sorbitan stearate; oil agents such as cetyl alcohol, cetostearyl alcohol, stearyl alcohol, vaseline, paraffin, liquid paraffin, cetyl palmitate, and octyl palmitate; guar gums such as xanthan gum, succinoglucan, hydroxypropyl guar gum, and cationized guar gum; thickening agents like celluloses such as carboxymethyl cellulose, hydroxyethyl cellulose, and cationized cellulose; moisturizing agents such as 1,3-BG, PG, DPG, and glycerol; chelating agents such as EDTA, EDTA-2Na, EDTA-4Na, and hydroxyethanediphosphonic acid; antiseptic agents such as paraben and methylisothiazolinone; solvents such as ethanol and isopropyl alcohol; perfumes; and the like. These can be arbitrarily combined and suitably blended in accordance with the needs.

The keratin fiber dye of the present invention may be either of one-agent type or of multiple-agent type. In the present invention, even if the oxidation dye and the oxidase are mixed with each other, color is not generated when oxygen is absent. Therefore, the dye may be of one-agent type when the dye is stored under anaerobic conditions, and the use thereof is easy. Also, the dye may be of multiple-agent type in which the electrostatic repulsion inhibitor and the oxidase are separately contained, such as of a two-agent type made of a first agent containing the electrostatic repulsion inhibitor and a second agent containing the oxidation dye and the oxidase, or of a two-agent type made of a first agent containing the electrostatic repulsion inhibitor and the oxidation dye and a second agent containing the oxidase. The dye may be of a two-agent type made of a first agent containing the electrostatic repulsion inhibitor and the oxidase and a second agent containing the oxidation dye. Further, the dye may be of a three-agent type made of a first agent containing the electrostatic repulsion inhibitor, a second agent containing the oxidation dye, and a third agent containing the oxidase.

The agent type may be an arbitrary agent type such as a liquid type, an emulsion type, a cream type, a gel type, a foam type, and an aerosol type. Also, any container can be adopted such as a bag, a bottle, a pumping-type container, a tube, and a spray can.

In order to dye the keratin fibers by using the keratin fiber dye described above, the keratin fibers are treated with the electrostatic repulsion inhibitor, or an agent containing at least the electrostatic repulsion inhibitor and the oxidase is used, or both of these are performed in combination.

A method for adding the electrostatic repulsion inhibitor to the agent containing the oxidase is not particularly limited as long as part or whole of the electric charge of the oxidase is neutralized by the electrostatic repulsion inhibitor. For example, the electrostatic repulsion inhibitor should be added to an enzyme liquid containing the oxidase, and the resulting liquid should be homogeneously stirred and left to stand for a predetermined period of time.

As described above, at the suitable pH range of the oxidase, the oxidase is negatively charged, and the electrostatic repulsion inhibitor is positively charged. Therefore, it is supposed that, by the above operation, the oxidase molecules floating in the enzyme liquid and the electrostatic repulsion inhibitor molecules are electrostatically adsorbed to form a complex. As a result, the electric charge of the complex as a whole is approximately zero or is slightly positive or negative, though the electric charge depends on the amount of the electrostatic repulsion inhibitor.

Also, a method for treating the keratin fibers with the electrostatic repulsion inhibitor is not particularly limited as long as part or whole of the electric charge on the surface of the keratin fibers is neutralized. An example of the above method may include a method in which a solution of the electrostatic repulsion inhibitor is sprayed and applied to the keratin fibers and, after a surplus solution is wiped off with a towel or the like, a part or whole of the solvent is volatilized in accordance with the needs. This allows the electrostatic repulsion inhibitor to be electrostatically adsorbed onto the keratin fibers, so that the surface of the keratin fibers is covered with the electrostatic repulsion inhibitor to form a complex. As a result, the electric charge of the complex as a whole is approximately zero or is slightly positive or negative, though the electric charge depends on the amount of the electrostatic repulsion inhibitor.

When both of the oxidase and the keratin fibers are positively charged in the complex as a whole by adding an excessive amount of the electrostatic repulsion inhibitor to the agent containing the oxidase and treating the keratin fibers with the excessive amount of the electrostatic repulsion inhibitor, the oxidase and the keratin fibers on the contrary repulse each other electrostatically, and thus the dyeing properties may be deteriorated. Therefore, in the case of adding the electrostatic repulsion inhibitor to the agent containing the oxidase and treating the keratin fibers with the electrostatic repulsion inhibitor, the amount of the electrostatic repulsion inhibitor is adjusted so that the amount of the electrostatic repulsion inhibitor for treatment may not become excessive, preferably so that the electric charge of the complex of the oxidase and the electrostatic repulsion inhibitor and the complex of the keratin fibers and the electrostatic repulsion inhibitor may become zero.

In the case of either the electrostatic repulsion inhibitor is blended with the agent containing the oxidase or the keratin fibers are treated with the electrostatic repulsion inhibitor, either one of the oxidase and the keratin fibers may be positively charged in the complex as a whole by treating with a large amount of the electrostatic repulsion inhibitor to thereby generate an electrostatic attractive force between the oxidase and the keratin fibers because one of the two is untreated and negatively charged. Therefore, the oxidase is adsorbed onto the keratin fibers, whereby the keratin fibers are advantageously more vividly dyed.

The complex as a whole can be more strongly charged to be positive, and the keratin fibers can be more vividly dyed by treating the keratin fibers with the electrostatic repulsion inhibitor rather than by adding the electrostatic repulsion inhibitor to the agent containing the oxidase. Therefore, it is preferable that only the keratin fibers are treated with the electrostatic repulsion inhibitor.

### EXAMPLES

Hereafter, the present invention will be described in further detail by way of Examples and Comparative Examples; however, it goes without saying that the present invention is in no way limited thereby. Here, in the following description, % means % by weight.

### Evaluation of electrostatic repulsion inhibitor

First, after a hair bundle (a) of human white hair (100%) (type number: BM-W-A, distributor: Beaulax Co., Ltd.) was immersed into a 0.5% solution of an electrostatic repulsion inhibitor to be evaluated for 5 minutes at room temperature (23.5°C), the hair bundle was washed with flowing water of 40°C for 30 seconds, followed by air-drying to obtain a treated hair bundle (b). The kind and isoelectric point of the electrostatic repulsion inhibitor to be evaluated, and the pH of the solution are shown in Table 1.

Meanwhile, a 0.1 mol/L citric acid solution and 0.2 mol/L disodium hydrogenphosphate were mixed at a ratio of 6.5 : 43.6 to prepare a buffer solution having a pH value of 6.81, and 0.5% basic blue 99 was blended into this buffer solution to prepare a dyeing liquid.

After the above treated hair bundle (b) that had been treated with the electrostatic repulsion inhibitor was immersed into 10 ml of this dyeing liquid for 10 minutes at room temperature (23.5°C) (bath ratio of 1:10), the hair bundle was washed with flowing water of 40°C for about 30 seconds, followed by air-drying to obtain a dyed hair bundle (c1). With respect to the obtained dyed hair bundle (c1) and the human white hair bundle (a), a color difference A between the dyed hair bundle (c1) and the untreated human white hair bundle (a) in the Lab color representation system was measured by using a spectrocolorimeter CM-2600d manufactured by KONICA MINOLTA, INC.

With respect to the untreated human white hair bundle (a) that had not been treated with the electrostatic repulsion inhibitor, the hair bundle was dyed in the same manner as described above to obtain a dyed hair bundle (c2), and a color difference B between the dyed hair bundle (c2) and the untreated human white hair bundle (a) in the Lab color representation system was measured.

The color difference A and the color difference B measured with respect to each electrostatic repulsion inhibitor, as well as a difference between the color difference A and the color difference B (color difference B - color difference A), are shown in Table 1.

**[Table 1]**

| Electric repulsion inhibitor | pH of solution | Color difference A | Color difference B | Color difference B-Color difference A |
|---|---|---|---|---|
| PA1 | 6.65 | 26.34 | 29.68 | 3.34 |
| *ε*-polylysine | 9.69 | 27.32 | 29.68 | 2.37 |
| Polyquaternium-2 | 7.20 | 26.19 | 29.68 | 3.49 |
| Polyquaternium-7 | 6.82 | 29.41 | 29.68 | 0.27 |
| Polyquaternium-72 | 5.47 | 28.59 | 29.68 | 1.09 |

### Example 1

A two-agent type keratin fiber dye made of a first agent containing an electrostatic repulsion inhibitor and a second agent containing 5,6-dihydroxyindole as a dye intermediate and an oxidase was prepared.

The first agent is a 0.5% aqueous solution of poly[oxyethylene(dimethylimino)propyl(dimethylimino)ethylene] (PA1, obtained from Nicca Chemical Co., Ltd.) (pH of 6.65) as the electrostatic repulsion inhibitor.

The second agent is obtained in such a manner that purified water is added to a mixture containing 2 g of polyoxyethylene hardened castor oil, 1 g of lactic acid, 0.9 g of monoethanolamine, and 0.3 g of 5,6-dihydroxyindole in an anaerobic environment so that a total weight would be 65 g, and bilirubin oxidase manufactured by Amano Enzyme Inc. (product name: BO-3, isoelectric point: 4.1, concentration at the time of use: 2 units/g) is added thereto as the oxidase. The pH value of the second agent is 7.3.

### Example 2

A keratin fiber dye was obtained in the same manner as in Example 1 except that the first agent was changed to a 0.5% aqueous solution of ε-polylysine (manufactured by Ichimaru Pharcos Co., Ltd., product name: POLYLYSINE 10) (pH of 9.69).

### Example 3

A keratin fiber dye was obtained in the same manner as in Example 1 except that the first agent was changed to a 0.5% aqueous solution of polyquaternium-2 (manufactured by Sigma-Aldrich Japan G. K. Co., Ltd., trade name:
poly[bis(2-chloroethyl)ether-alt-1.3-bis[3-(dimethylamino)propyl]urea], quaternized product solution) (pH of 7.2).

### Example 4

A keratin fiber dye was obtained in the same manner as in Example 1 except that the first agent was changed to a 0.5% aqueous solution of polyquaternium-7 (manufactured by Senka Corporation, trade name: COSMUAT VH) (pH of 6.82).

### Example 5

A keratin fiber dye was obtained in the same manner as in Example 1 except that the first agent was changed to a 0.5% aqueous solution of polyquaternium-72 (manufactured by Croda Japan KK., trade name: MiruStyle CP) (pH of 5.47).

### Examples 6 and 7 (reference)

Keratin fiber dyes were obtained in the same manner as in Examples 1 and 3 except that laccase (manufactured by Amano Enzyme Inc., product name: M120, isoelectric point: 3.0) was used as the oxidase of the second agent in place of the bilirubin oxidase.

With regard to Examples 1 to 7 above, the electrostatic repulsion inhibitor used in the first agent and the oxidase used in the second agent are shown together in Table 2.

**[Table 2]**

| Keratin fiber dye | First agent | Second agent * |
|---|---|---|
| | Electric repulsion inhibitor | Oxidase |
| Example 1 | PA1 | Bilirubin oxidase |
| Example 2 | *ε*-polylysine | Bilirubin oxidase |
| Example 3 | Polyquaternium-2 | Bilirubin oxidase |
| Example 4 | Polyquaternium-7 | Bilirubin oxidase |
| Example 5 | Polyquaternium-72 | Bilirubin oxidase |
| Example 6 | PA1 | Laccase |
| Example 7 | Polyquaternium-2 | Laccase |

| | | |
|---|---|---|
| * Dye: Containing 5,6-dihydroxyindole | | |

### Examples 8 to 14 (hair dyeing 1) (Examples 13 and 14: reference)

By using the keratin fiber dye of each of Examples 1 to 7 described above, a dyeing test was performed on a hair bundle of 1 g of human white hair (100%) (type number: BM-W-A, distributor: Beaulax Co., Ltd.).

First, as pretreatment, the hair bundle was washed with flowing water of about 40°C for 30 seconds, washed with 1% SDS (sodium dodecylsulfate) liquid by hand for 1 minute, washed with flowing water of about 40°C for 30 seconds again to wash away the SDS liquid, and thereafter subjected to air-drying.

Subsequently, 10 ml of the first agent was taken, and the hair bundle subjected to the above air-drying was immersed thereinto for 5 minutes at room temperature (23.5°C), thereafter washed with flowing water of 40°C for 30 seconds, and then subjected to air-drying.

Subsequently, 2.0 g of the second agent was weighed and applied onto the hair bundle with a brush. The hair bundle was widened to form a fan shape so that the second agent could be applied onto the hair bundle as uniformly as possible.

After the application, the resultant was left to stand for 10 minutes at room temperature (23.5°C). Thereafter, the hair bundle was turned over, and the second agent was spread uniformly with a brush in a similar manner. Further, the resultant was left to stand for 10 minutes at room temperature.

Thereafter, the hair bundle was washed with flowing water of about 40°C for 30 seconds to wash away the keratin fiber dye. Then, after 1% SDS liquid was applied and foamed by combing for 20 times with a comb, the 1% SDS liquid was washed away by combing for 20 times with a comb while allowing water of about 40°C to flow. After the washing, the moisture was wiped away with a towel, and dried.

A color difference ΔE between the dyed hair bundle subjected to the drying and an untreated human white hair bundle in the Lab color representation system was measured by using a spectrocolorimeter (manufactured by KONICA MINOLTA, INC, CM-2600d). The dyes used and the measurement results are shown in Table 3.

### Examples 15 to 21 (hair dyeing 2) (Examples 20 and 21: reference)

Hair bundles were dyed in the same manner as in Examples 8 to 14 except that the treatment with the first agent was changed to the following method, and a color difference ΔE between each of the dyed hair bundles and an untreated human white hair bundle was measured. The dyes used and the measurement results are shown in Table 3.

The treatment with the first agent was performed in such a manner that the first agent was applied so that the bath ratio would be 5:1 to the hair bundle that had been subjected to the pretreatment (0.2 g relative to 1 g of hair bundle), followed by air-drying.

### Comparative Example 1

A hair bundle was dyed in the same manner as in Example 8 except that the treatment with the first agent was not performed, and a color difference ΔE in the Lab color representation system was measured. The measurement result is shown in Table 3.

As will be clear from Examples 8 to 14 and Examples 15 to 21 of Table 3, the effect produced by the treatment with the electrostatic repulsion inhibitor is recognized irrespective of whether the first agent (electrostatic repulsion inhibitor) is washed away or not. There is an electrostatic repulsion inhibitor that gives a difference in the effect, so that the electrostatic repulsion inhibitor may be used by suitably selecting whether the electrostatic repulsion inhibitor is to be washed away or not.

**[Table 3]**

| | Keratin fiber dye | Color difference Δ E |
|---|---|---|
| Example 8 | Example 1 | 26.71 |
| Example 9 | Example 2 | 21.65 |
| Example 10 | Example 3 | 21.41 |
| Example 11 | Example 4 | 26.65 |
| Example 12 | Example 5 | 15.75 |
| Example 13 | Example 6 | 19.52 |
| Example 14 | Example 7 | 19.04 |
| Example 15 | Example 1 | 26.66 |
| Example 16 | Example 2 | 22.85 |
| Example 17 | Example 3 | 25.54 |
| Example 18 | Example 4 | 22.29 |
| Example 19 | Example 5 | 20.93 |
| Example 20 | Example 6 | 19.01 |
| Example 21 | Example 7 | 21.48 |
| Comp. Example 1 | (First agent is absent.) | 12.41 |

### Example 22

A hair bundle was dyed in the same manner as in Example 8 except that PA1 was also blended in the second agent so that the concentration would be 0.5%, and a color difference ΔE in the Lab color representation system was measured. The measurement result is shown in Table 4.

### Example 23

A hair bundle was dyed in the same manner as in Example 22 except that the treatment with the first agent was not performed, and a color difference ΔE in the Lab color representation system was measured. The measurement result is shown in Table 4.

### Comparative Example 2

A hair bundle was dyed in the same manner as in Example 8 except that the treatment with the first agent was not performed, and a color difference ΔE in the Lab color representation system was measured. The measurement result is shown in Table 4.

**[Table 4]**

| | Hair bundle (First agent) | Oxidase (Second agent *) | Color difference ΔE |
|---|---|---|---|
| Example 22 | With treatment | Blended | 20.5 |
| Example 23 | Without treatment | Blended | 21.6 |
| Example 8 | With treatment | Unblended | 26.7 |
| Comp. Example 2 | Without treatment | Unblended | 12.4 |

| | | | |
|---|---|---|---|
| * Dye: Containing 5,6-dihydroxyindole | | | |

In the column of "hair bundle (first agent)" in Table 4, the case where the hair bundle was treated with the electrostatic repulsion inhibitor of the first agent is denoted as "with treatment", and the case where the hair bundle was not treated is denoted as "without treatment". In the column of "oxidase (second agent)" in Table 4, the case where the electrostatic repulsion inhibitor was blended into the second agent containing the dye and the oxidase is denoted as "blended", and the case where the electrostatic repulsion inhibitor is not blended is denoted as "unblended". For convenience of comparison, the results of Example 8 were shown together.

As shown in Table 4, it is understood that Example 22 in which the hair bundle (keratin fibers) was treated with the electrostatic repulsion inhibitor of the first agent and in which the electrostatic repulsion inhibitor was blended into the second agent containing the dye and the oxidase gives a color difference ΔE larger than that of Comparative Example 2 in which the electrostatic repulsion inhibitor was not blended, thereby confirming that Example 22 is excellent in dyeing properties. This is supposed to be because the electrostatic repulsive force between the hair bundle and the oxidase was almost negated by the electrostatic repulsion inhibitor.

It is understood that Example 23 in which the hair bundle was not treated with the electrostatic repulsion inhibitor of the first agent and in which the electrostatic repulsion inhibitor was blended into the second agent containing the dye and the oxidase and Example 8 in which the hair bundle was treated with the electrostatic repulsion inhibitor of the first agent and in which the electrostatic repulsion inhibitor was not blended into the second agent give a color difference ΔE further larger than that of Example 22 in which the hair bundle was treated with the electrostatic repulsion inhibitor of the first agent and in which the electrostatic repulsion inhibitor was blended into the second agent, thereby confirming that Example 23 and Example 8 are more excellent in dyeing properties. This is supposed to be due to the following reason: that is, when the hair bundle is treated with the electrostatic repulsion inhibitor and the electrostatic repulsion inhibitor is blended into the second agent, both the hair bundle and the oxidase are positively charged, so that an electrostatic repulsive force is generated though weakly. In contrast, when only one of the two is treated, one of the two is positively charged, and the other one is negatively charged, so that an electrostatic attractive force is generated between the hair bundle and the oxidase.

Further, it is understood that Example 8 in which the hair bundle was only treated with the electrostatic repulsion inhibitor gives a color difference ΔE further larger than that of Example 23 in which the electrostatic repulsion inhibitor was blended into the second agent containing the dye and the oxidase, thereby confirming that Example 8 is the most excellent in dyeing properties. This is supposed to be because, even when the same electrostatic repulsion inhibitor is used, the hair bundle is more liable to be positively charged than the oxidase, so that a stronger electrostatic attractive force is generated.

### Example 24

A two-agent type keratin fiber dye made of a first agent containing an electrostatic repulsion inhibitor and a second agent containing paraphenylenediamine as a dye intermediate and an oxidase was prepared.

In the same manner as in Example 1, the first agent is a 0.5% aqueous solution of poly[oxyethylene(dimethylimino)propyl(dimethylimino)ethylene] (PA1, obtained from Nicca Chemical Co., Ltd.) (pH of 6.65) as the electrostatic repulsion inhibitor.

The second agent is obtained in such a manner that purified water is added to a mixture containing 2 g of polyoxyethylene hardened castor oil, 1 g of lactic acid, a suitable amount of monoethanolamine (adjusted at around pH of 6), and 0.5 g of paraphenylenediamine in an anaerobic environment so that a total weight would be 65 g, and bilirubin oxidase manufactured by Amano Enzyme Inc. (product name: BO-3, isoelectric point: 4.1, concentration at the time of use: 2 units/g) is added thereto as the oxidase. The final pH value of the second agent is 6.08.

### Example 25

A keratin fiber dye was obtained in the same manner as in Example 24 except that, in the second agent, the pH value was adjusted at around 7 with monoethanolamine. The final pH value of the second agent is 7.02.

### Examples 26 and 27

By using the keratin fiber dye of Examples 24 and 25 described above, hair bundles were dyed in the same manner as in Examples 8 to 14 (hair dyeing 1), and a color difference ΔE in the Lab color representation system was measured. The measurement results are shown in Table 5.

### Comparative Examples 3 and 4

Hair bundles were dyed in the same manner as in Examples 26 and 27 except that the treatment with the first agent was not performed, and a color difference ΔE in the Lab color representation system was measured. The measurement results are shown in Table 5.

**[Table 5]**

| | Keratin fiber dye * | Color difference Δ E |
|---|---|---|
| Example 26 | Example 24 | 47.3 |
| Example 27 | Example 25 | 49.1 |
| Comp. Example 3 | Second agent of Example 24 | 46.2 |
| Comp. Example 4 | Second agent of Example 25 | 48.6 |

| | | |
|---|---|---|
| * Dye: Containing paraphenylenediamine | | |

As shown in Table 5, it is understood that the dyeing properties are enhanced even when the kind of the oxidation dye is changed.

### INDUSTRIAL APPLICABILITY

The method for dyeing keratin fibers and the keratin fiber dye according to the present invention have excellent dyeing properties due to the following reason: because the electrostatic repulsion inhibitor is blended, the electrostatic repulsion between the oxidase and the keratin fibers is negated or reduced, and preferably in the state where the oxidase adheres to the keratin fibers, the oxidation dye is oxidized to generate color and adsorbed onto the keratin fibers. The present invention provides less damage and irritation to the hair and scalp.

## Claims

1. A method for dyeing keratin fibers using an electrostatic repulsion inhibitor having a property of being positively charged under the pH conditions during the dyeing, an oxidation dye and an oxidase that can oxidize the oxidation dye and under pH conditions where both the oxidase and the keratin fibers are negatively charged, which comprises the step of:
before dyeing, blending the electrostatic repulsion inhibitor with the agent containing the oxidase or treating the keratin fibers with the electrostatic repulsion inhibitor,**characterized in that** the electrostatic repulsion inhibitor is at least one kind selected from the group consisting of ε-polylysine, polyquaternium-2, polyquaternium-7, polyquaternium-72 and poly[oxyethylene(dimethylimino)propyl(dimethylimino)-ethylene], and
the oxidase is bilirubin oxidase (BOD).

2. The method for dyeing keratin fibers according to claim 1, wherein an agent containing at least the electrostatic repulsion inhibitor and the oxidase is used.

3. The method for dyeing keratin fibers according to claim 1, wherein, before dyeing, the keratin fibers are treated with the electrostatic repulsion inhibitor, and wherein an agent containing at least the oxidation dye and the oxidase is used.

4. A keratin fiber dye comprising an electrostatic repulsion inhibitor, an oxidation dye, and an oxidase that can oxidize the oxidation dye,
wherein, under pH conditions during dyeing, the oxidase has a property of being negatively charged, and
wherein the electrostatic repulsion inhibitor has a property of being positively charged, **characterized in that** the electrostatic repulsion inhibitor is at least one kind selected from the group consisting of ε-polylysine, polyquaternium-2, polyquaternium-7, polyquaternium-72 and poly[oxyethylene(dimethylimino)propyl(dimethylimino)-ethylene], and
the oxidase is bilirubin oxidase (BOD).

5. A kit of parts which is a keratin fiber dye comprising an electrostatic repulsion inhibitor, an oxidation dye, and an oxidase that can oxidize the oxidation dye,
wherein the components of the kit are arranged as follows:
- a two-agent type where the electrostatic repulsion inhibitor is contained separately from the oxidation dye and the oxidase,
- a two-agent type where the electrostatic repulsion inhibitor and the oxidation dye are contained separately from the oxidase,
- a two-agent type where the electrostatic repulsion inhibitor and the oxidase are contained separately from the oxidation dye, or
- a three-agent type where the electrostatic repulsion inhibitor, the oxidation dye, and the oxidase are contained separately,
wherein, under pH conditions during dyeing, the oxidase has a property of being negatively charged, and
wherein the electrostatic repulsion inhibitor has a property of being positively charged, **characterized in that** the electrostatic repulsion inhibitor is at least one kind selected from the group consisting of ε-polylysine, polyquaternium-2, polyquaternium-7, polyquaternium-72 and poly[oxyethylene(dimethylimino)propyl(dimethylimino)-ethylene], and
the oxidase is bilirubin oxidase (BOD).

6. The kit of parts according to claim 5, which is a two-agent type where the electrostatic repulsion inhibitor is contained separately from the oxidation dye and the oxidase.

7. The kit of parts according to claim 5, which is a two-agent type where the electrostatic repulsion inhibitor and the oxidation dye are contained separately from the oxidase.

8. The kit of parts according to claim 5, which is a two-agent type where the electrostatic repulsion inhibitor and the oxidase are contained separately from the oxidation dye.

9. The kit of parts according to claim 5, which is a three-agent type where the electrostatic repulsion inhibitor, the oxidation dye, and the oxidase are contained separately.

## Patentansprüche

1. Ein Verfahren zum Färben von Keratinfasern unter Verwendung eines elektrostatischen Abstoßungsinhibitors mit der Eigenschaft, unter den pH-Bedingungen während des Färbens positiv geladen zu sein, eines Oxidationsfarbstoffs und einer Oxidase, die den Oxidationsfarbstoff oxidieren kann, und unter pH-Bedingungen, bei denen sowohl die Oxidase als auch die Keratinfasern negativ geladen sind, umfassend den Schritt von:
vor dem Färben, Mischen des elektrostatischen Abstoßungsinhibitors mit dem die Oxidase enthaltenden Mittel oder Behandeln der Keratinfasern mit dem elektrostatischen Abstoßungsinhibitor, **dadurch gekennzeichnet, dass** der elektrostatische Abstoßungsinhibitor mindestens eine Art ist, ausgewählt aus der Gruppe bestehend aus ε-Polylysin, Polyquaternium-2, Polyquaternium-7, Polyquaternium-72 und Poly[oxyethylen(dimethylimino)propyl(dimethylimino)ethylen], und
die Oxidase eine Bilirubinoxidase (BOD) ist.

2. Das Verfahren zum Färben von Keratinfasern gemäß Anspruch 1, wobei ein Mittel, welches mindestens den elektrostatischen Abstoßungsinhibitor und die Oxidase enthält, verwendet wird.

3. Das Verfahren zum Färben von Keratinfasern gemäß Anspruch 1, wobei die Keratinfasern vor dem Färben mit dem elektrostatischen Abstoßungsinhibitor behandelt werden, und wobei ein Mittel verwendet wird, das mindestens den Oxidationsfarbstoff und die Oxidase enthält.

4. Ein Keratinfaserfarbstoff, umfassend einen elektrostatischen Abstoßungsinhibitor, einen Oxidationsfarbstoff und eine Oxidase, die den Oxidationsfarbstoff oxidieren kann, wobei die Oxidase unter pH-Bedingungen während des Färbens die Eigenschaft hat, negativ geladen zu sein, und
wobei der elektrostatische Abstoßungsinhibitor die Eigenschaft hat, positiv geladen zu sein,
**dadurch gekennzeichnet, dass** der elektrostatische Abstoßungsinhibitor mindestens eine Art ist, ausgewählt aus der Gruppe bestehend aus ε-Polylysin, Polyquaternium-2, Polyquaternium-7, Polyquaternium-72 und Poly[oxyethylen(dimethylimino)propyl(dimethylimino)ethylen], und
die Oxidase eine Bilirubinoxidase (BOD) ist.

5. Ein Teile-Kit, das ein Keratinfaserfarbstoff ist, umfassend einen elektrostatischen Abstoßungsinhibitor, einen Oxidationsfarbstoff und eine Oxidase, die den Oxidationsfarbstoff oxidieren kann,
wobei die Komponenten des Kits wie folgt angeordnet sind:
- zweiteilig, wobei der elektrostatische Abstoßungsinhibitor getrennt vom Oxidationsfarbstoff und der Oxidase enthalten ist,
- zweiteilig, wobei der elektrostatische Abstoßungsinhibitor und der Oxidationsfarbstoff getrennt von der Oxidase enthalten sind,
- zweiteilig, wobei der elektrostatische Abstoßungsinhibitor und die Oxidase getrennt vom Oxidationsfarbstoff enthalten sind, oder
- dreiteilig, wobei der elektrostatische Abstoßungsinhibitor, der Oxidationsfarbstoff und die Oxidase getrennt voneinander enthalten sind,
wobei die Oxidase unter pH-Bedingungen während des Färbens die Eigenschaft hat, negativ geladen zu sein, und
wobei der elektrostatische Abstoßungsinhibitor die Eigenschaft hat, positiv geladen zu sein,
**dadurch gekennzeichnet, dass** der elektrostatische Abstoßungsinhibitor mindestens eine Art ist, ausgewählt aus der Gruppe bestehend aus ε-Polylysin, Polyquaternium-2, Polyquaternium-7, Polyquaternium-72 und Poly[oxyethylen(dimethylimino)propyl(dimethylimino)ethylen], und
die Oxidase eine Bilirubinoxidase (BOD) ist.

6. Das Teile-Kit gemäß Anspruch 5, das zweiteilig ist, wobei der elektrostatische Abstoßungsinhibitor getrennt von dem Oxidationsfarbstoff und der Oxidase enthalten ist.

7. Das Teile-Kit gemäß Anspruch 5, das zweiteilig ist, wobei der elektrostatische Abstoßungsinhibitor und der Oxidationsfarbstoff getrennt von der Oxidase enthalten sind.

8. Das Teile-Kit gemäß Anspruch 5, das zweiteilig ist, wobei der elektrostatische Abstoßungsinhibitor und die Oxidase getrennt vom Oxidationsfarbstoff enthalten sind.

9. Das Teile-Kit gemäß Anspruch 5, das dreiteilig ist, wobei der elektrostatische Abstoßungsinhibitor, der Oxidationsfarbstoff und die Oxidase getrennt enthalten sind.

## Revendications

1. Méthode pour teindre des fibres de kératine utilisant un inhibiteur de répulsion électrostatique ayant pour propriété d'être chargé positivement dans les conditions de pH durant la teinture, un colorant d'oxydation et une oxydase qui peut oxyder le colorant d'oxydation et dans des conditions de pH où tant l'oxydase que les fibres de kératine sont chargées négativement, qui comprend l'étape suivante :
avant la teinture, le mélange de l'inhibiteur de répulsion électrostatique avec l'agent contenant l'oxydase ou le traitement des fibres de kératine avec l'inhibiteur de répulsion électrostatique, **caractérisée en ce que** l'inhibiteur de répulsion électrostatique est d'au moins un type choisi dans le groupe constitué par l'ε-polylysine, le polyquaternium-2, le polyquaternium-7, le polyquaternium-72 et le poly[oxyéthylène(diméthylimino)propyl(diméthylimino)éthylène], et l'oxydase est la bilirubine oxydase (BOD).

2. Méthode pour teindre des fibres de kératine selon la revendication 1, dans laquelle un agent contenant au moins l'inhibiteur de répulsion électrostatique et l'oxydase est utilisé.

3. Méthode pour teindre des fibres de kératine selon la revendication 1, dans laquelle, avant la teinture, les fibres de kératine sont traitées avec l'inhibiteur de répulsion électrostatique, et dans laquelle un agent contenant au moins le colorant d'oxydation et l'oxydase est utilisé.

4. Colorant pour fibres de kératine comprenant un inhibiteur de répulsion électrostatique, un colorant d'oxydation, et une oxydase qui peut oxyder le colorant d'oxydation, dans lequel, dans les conditions de pH durant la teinture, l'oxydase a pour propriété d'être chargée négativement, et
dans lequel l'inhibiteur de répulsion électrostatique a pour propriété d'être chargé positivement, **caractérisé en ce que** l'inhibiteur de répulsion électrostatique est d'au moins un type choisi dans le groupe constitué par l'ε-polylysine, le polyquaternium-2, le polyquaternium-7, le polyquaternium-72 et le poly[oxyéthylène(diméthylimino)propyl(diméthylimino)éthylène], et
l'oxydase est la bilirubine oxydase (BOD).

5. Kit de pièces qui est un colorant pour fibres de kératine comprenant un inhibiteur de répulsion électrostatique, un colorant d'oxydation, et une oxydase qui peut oxyder le colorant d'oxydation,
dans lequel les composants du kit sont disposés comme suit :
- un type à deux agents où l'inhibiteur de répulsion électrostatique est contenu séparément du colorant d'oxydation et de l'oxydase,
- un type à deux agents où l'inhibiteur de répulsion électrostatique et le colorant d'oxydation sont contenus séparément de l'oxydase,
- un type à deux agents où l'inhibiteur de répulsion électrostatique et l'oxydase sont contenus séparément du colorant d'oxydation, ou
- un type à trois agents où l'inhibiteur de répulsion électrostatique, le colorant d'oxydation et l'oxydase sont contenus séparément,
dans lequel, dans les conditions de pH durant la teinture, l'oxydase a pour propriété d'être chargée négativement et,
dans lequel l'inhibiteur de répulsion électrostatique a pour propriété d'être chargé positivement,
**caractérisé en ce que** l'inhibiteur de répulsion électrostatique est d'au moins un type choisi dans le groupe constitué par l'ε-polylysine, le polyquaternium-2, le polyquaternium-7, le polyquaternium-72 et le poly[oxyéthylène(diméthylimino)propyl(diméthylimino)éthylène], et
l'oxydase est la bilirubine oxydase (BOD).

6. Kit de pièces selon la revendication 5, qui est un type à deux agents où l'inhibiteur de répulsion électrostatique est contenu séparément du colorant d'oxydation et de l'oxydase.

7. Kit de pièces selon la revendication 5, qui est un type à deux agents où l'inhibiteur de répulsion électrostatique et le colorant d'oxydation sont contenus séparément de l'oxydase.

8. Kit de pièces selon la revendication 5, qui est un type à deux agents où l'inhibiteur de répulsion électrostatique et l'oxydase sont contenus séparément du colorant par oxydation.

9. Kit de pièces selon la revendication 5, qui est un type à trois agents où l'inhibiteur de répulsion électrostatique, le colorant d'oxydation et l'oxydase sont contenus séparément.
